# EUROPÄISCHE PATENTSCHRIFT

(11) **EP 0 591 248 B1**
(45) Veröffentlichungstag und Bekanntmachung des Hinweises auf die Patenterteilung: **30.08.1995**
(21) Anmeldenummer: 92911090.6
(22) Anmeldetag: 09.06.1992
(51) Int. Cl.: A61F 13/15

(54) **WEGWERFWINDEL**
DISPOSABLE DIAPERS
COUCHE A JETER

(30) Priorität: 28.06.1991 DE 4121419
(43) Veröffentlichungstag der Anmeldung: 13.04.1994
(73) Patentinhaber: Paul Hartmann Aktiengesellschaft, D-89522 Heidenheim (DE)
(72) Erfinder:
(74) Vertreter: Becker, Maria, Dipl.-Phys.
(86) Internationale Anmeldenummer: DE9200486
(87) Internationale Veröffentlichungsnummer: WO9300059

(56) Entgegenhaltungen:
- EP-A- 0 109 126
- EP-A- 0 376 022
- EP-A- 0 454 105
- US-A- 4 735 622

## Beschreibung

Die Erfindung betrifft eine Wegwerfwindel mit einer gegenüber Flüssigkeit undurchlässigen Wäscheschutzfolie, einer gegenüber Flüssigkeit durchlässigen Vliesstoffabdeckung und einem zwischen der Wäscheschutzfolie und der Vliesstoffabdeckung sich befindenden Saugkörper, sowie einer die Aussenkontur bildenden Dichtungsleiste.

Es ist allgemein bekannt, daß derart ausgebildete Windeln, die zum einmaligen Gebrauch bestimmt sind, Körperausscheidungen von Kleinkindern und Inkontinenten aufnehmen, so daß diese Personen nach der Abgabe der Flüssigkeit kein Unbehagen empfinden, da der Saugkörper der Windel die Flüssigkeit vollständig in sich aufnimmt. Die von der Windel bedeckte Hautoberfläche dieser Personen ist nicht nur subjektiv, sondern auch objektiv trocken, da der Saugkörper die Flüssigkeit von der Hautoberfläche fernhält. Dies ist wünschenswert, damit die Haut von der abgesonderten Körperflüssigkeit nicht angegriffen wird, zumal in dem von der Windel eingeschlossenen Raum ein besonders günstiges Klima für Bakterien und Pilze herrscht. Jedoch hat sich herausgestellt, daß aufgrund der Annehnlichkeit die Benutzer dieser Windeln nach Abgabe der Körperausscheidungen, insbesondere heilbar Inkontinente und größere Kleinkinder, durch nichts dazu veranlaßt werden, die Toilette aufzusuchen, wenn sie einen Drang verspüren. Dies führt dazu, daß aufgrund des fehlenden Lerneffekts die Gewöhnung an den normalen Toilettengang entweder sehr spät oder bei krankhaft Inkontinenten gar nicht eintritt.

Der Erfindung liegt daher die Aufgabe zugrunde, eine Windel der eingangs genannten Art derart weiterzubilden, daß mit ihr ein Lerneffekt erzielt wird, der die inkontinenten Personen und Kinder dazu erzieht, die Toilette aufzusuchen.

Diese Aufgabe wird erfindungsgemäß dadurch gelöst, daß ein die Außenkontur der Dichtungsleisten seitlich zumindest teilweise überragende, hydrophiler Seitenklappen vorgesehen ist, der mit der gegenüber Flüssigkeit durchlässigen Vliesstoffabdeckung verbunden ist.

Zwar sind Seitenklappen allgemein bekannt (EP-A-0 376 022 EP 0 219 326), diese dienen jedoch dazu, die in die Windel ausgeschiedene Körperflüssigkeit sicher in der Windel zu halten, so daß sie an den Seitenrändern nicht austreten kann. Derartige Seitenklappen sind also als Dichtungsleisten ausgebildet.

Bei der erfindungsgemäßen Wegwerfwindel haben die Seitenklappen jedoch die Funktion, einen Teil der Körperflüssigkeit aus der Windel herauszuleiten. Mittels dieser Seitenklappen werden also die Dichtungsleisten umgangen. Die Ableitung von Körperflüssigkeit erfolgt dadurch, daß die Seitenklappen mit der Vliesstoffabdeckung, auf die die Körperflüssigkeit zuerst auftrifft, direkt verbunden ist. Es wird also sofort nach Abgabe der Körperausscheidung ein Teil der Flüssigkeit aus der Windel herausgeleitet. Die Geschwindigkeit hängt von der Saugfähigkeit der Seitenklappen ab. Sind die aus der Windel herausragenden Abschnitte der Seitenklappen mit Flüssigkeit benetzt, so tritt für den Inkontinenten dann ein unangenehmes Gefühl auf, wenn dieser benetzte Teil der Seitenklappen mit der Hautoberfläche in Berührung kommt. In der Regel verdunstet an den Seitenklappen ein Teil der Flüssigkeit, wodurch dieser sich aufgrund der Verdampfungsenthalpie abkühlt. Diese Kälte wird jedoch vom Benutzer als unangenehm empfunden. Allmählich wird dann das unangenehme Kälteempfinden mit der Flüssigkeitsabsonderung in Verbindung gebracht. Diesen Personen kann nun beigebracht werden, daß bei der Benutzung der Toilette der unangenehme Kälteeffekt nicht mehr auftritt, da keine Flüssigkeit mehr in die Windel abgesondert wird. Die erfindungsgemäße Windel weist somit den Vorteil auf, daß die von der Windel abgedeckte Hautoberfläche nicht von der abgesonderten Körperflüssigkeit angegriffen wird, da diese von der Hautoberfläche ferngehalten wird, die inkontinente Person aber trotzdem aufgrund des Kältegefühls angehalten wird, die Absonderung der Körperflüssigkeit zu kontrollieren.

Vorteilhaft überragen die hydrophilen Seitenklappen die Außenkontur der Dichtungsleisten im Bereich der Beinausschnitte. Hierdurch wird der Vorteil erzielt, daß die Seitenklappen, aie aufgrund der hydrophilen Eigenschaft die Feuchtigkeit aus dem Windelinnenraum absorbieren, im Schrittbereich an den Innenseiten der Oberschenkel anliegen. Diese Körperregion ist jedoch besonders empfindlich. Es werden bereits geringe Temperaturunterschiede gegenüber der Umgebung als unangenehm empfunden. Es können also schon die kleinsten, aus dem Windelraum aufgesogenen Flüssigkeitsmengen als störend empfunden werden. Da die Seitenklappen die Windel lediglich im Bereich der Beinausschnitte überragen, jedoch innerhalb der Windel über deren ganze Länge mit der Vliesstoffabdeckung verbunden sind, ist die Dochtwirkung und somit die Zeitdauer für das Aufsaugen eines Teils der Flüssigkeit unabhängig vom Ort, an dem die Flüssigkeit an die Windel abgegeben wird.

Bei einer anderen Ausführungsform ist vorgesehen, daß die Seitenklappen nur im Bereich der Beinausschnitte vorgesehen sind. Diese Seitenklappen erstrecken sich nicht über die ganze Länge der Windel, sondern sind lediglich in dem Bereich vorgesehen, wo hauptsächlich Körperflüssigkeit anfällt. Bei dieser Ausführungsform weisen die Seitenklappen eine geringere Länge auf und die Windel kann deshalb kostengünstiger hergestellt werden.

Bei einer weiteren Ausführungsform erstrecken sich die Seitenklappen über die ganze Länge der Windel. Dies hat den Vorteil, daß das Aufbringen der Seitenklappen einfach gestaltet wird, da es von einem endlosen Band abgerollt und auf die ebenfalls noch zu einem endlosen Band zusammenhängenden Windeln aufgelegt und mit diesen verschweißt und gemeinsam mit diesen abgetrennt werden kann.

Eine bevorzugte Ausgestaltung sieht vor, daß die Seitenklappen in einem Bereich mit der Vliesstoffabdeckung gegebenenfalls einstückig verbunden sind, in dem diese den Saugkörper überdeckt. Dies hat den Vorteil, daß die Flüssigkeitsabsorption der Seitenklappen nicht nur von der Vliesstoffabdeckung, sondern auch vom Saugkörper erfolgt. Wird bei einer derart ausgebildeten Windel die Flüssigkeit einseitig an die Vliesstoffabdeckung abgegeben, so gelangt sie dennoch an beide Seitenklappen, da die Flüssigkeit durch den Saugkörper gleichmäßig innerhalb der Windel und dadurch auch in den Bereich beider Seitenklappen verteilt wird.

Bei einer speziellen Ausführungsform ist vorgesehen, daß lediglich eine Seitenklappe an einer einzigen Seite der Windel angebracht ist.

Vorzugsweise sind die Seitenklappen gegenüber Flüssigkeiten absorbierend bzw. besitzen die Seitenklappen eine Dochtwirkung. Hierdurch wird die Flüssigkeit relativ schnell aus dem Windelinnenraum nach außen an die Hautoberfläche des Trägers geführt. Durch die Wahl des Materials, der Dichte oder eventueller Oberflächenbehandlungen kann die Sauggeschwindigkeit auf einen gewünschten Wertebereich eingestellt werden.

Eine Weiterbildung sieht vor, daß die Seitenklappen bei an den Körper angelegter Windel sich von der Windel weg erstrecken. Vorteilhaft liegen die Seitenklappen an der Innenseite des Schenkels des Trägers an bzw. berühren die Innenseite zumindest abschnittsweise.

Ein geeignetes Material für die Seitenklappen ist Viskose, wobei jedoch auch andere geeignete Materialien verwendet werden können.

Bevorzugt sind die Seitenklappen auf die Vliesstoffabdeckung aufgeklebt. Dies kann z.B. mittels eines Heiß- oder Schmelzklebers bereits bei der Herstellung der Windel erfolgen.

Weitere Vorteile, Merkmale und Einzelheiten der Erfindung ergeben sich aus der nachfolgenden Beschreibung, in der unter Bezugnahme auf die Zeichnung besonders bevorzugte Ausführungsbeispiele der Erfindung im einzelnen erläutert sind.

In der Zeichnung zeigen:
- Figur 1: eine Draufsicht auf eine Hälfte einer Windel mit im Beinausschnitt angeordneter Seitenklappe;
- Figur 2: eine Draufsicht auf eine Hälfte einer Windel mit Sicht über die sich über die ganze Länge der Windel erstreckender Seitenklappe; und
- Figur 3: einen Querschnitt III-III entlang der Mittellinie gemäß Fig. 1 u. 2.

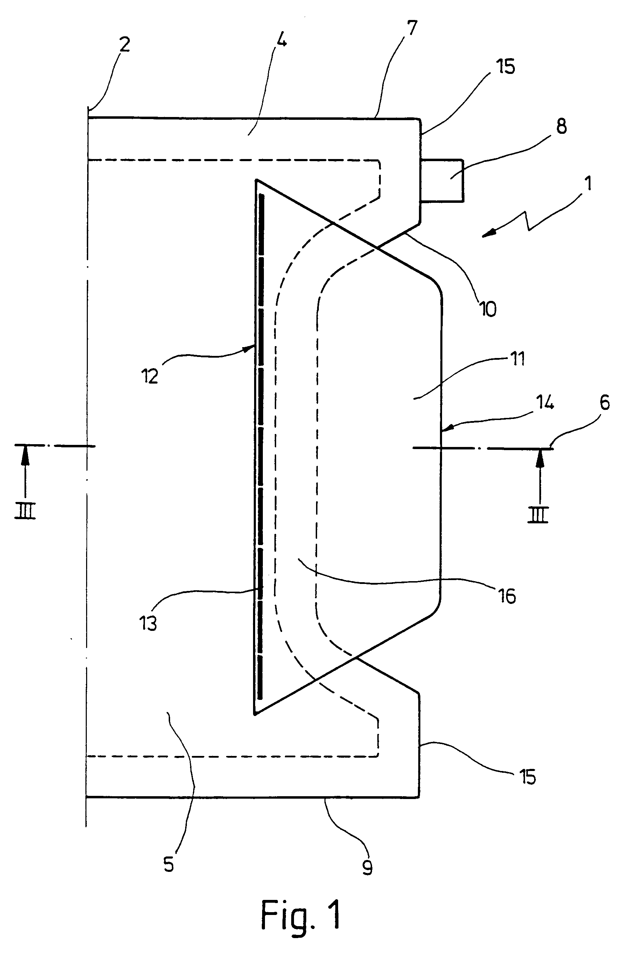

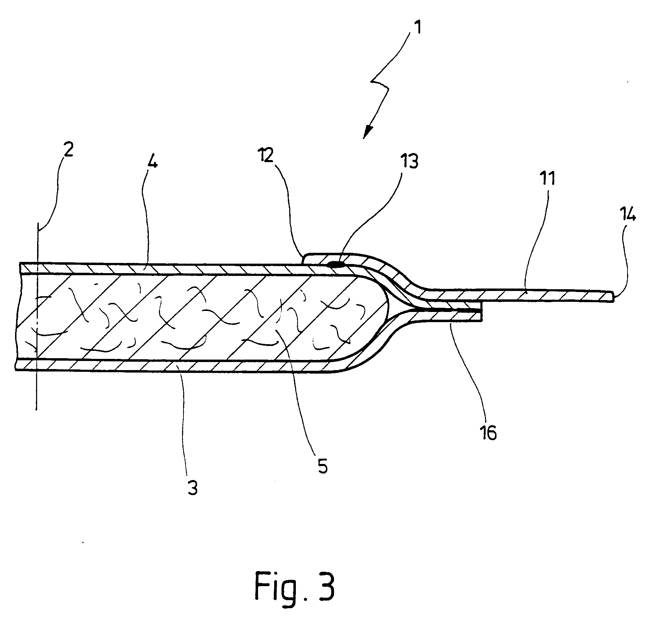

In der Figur 1 ist eine insgesamt mit 1 bezeichnete Windel in Draufsicht dargestellt, wobei lediglich die rechts von der Längsmittellinie 2 liegende Hälfte dargestellt ist. Diese Windel 1 ist an ihrer Unterseite mit einer Wäscheschutzfolie 3 versehen und weist an ihrer Oberseite eine Vliesstoffabdeckung 4 auf, was in der Fig. 3 deutlich dargestellt ist. Zwischen der Wäscheschutzfolie 3 und der Vliesstoffabdeckung 4 befindet sich ein Saugkörper 5, der in die Windel 1 abgegebene Flüssigkeit vollständig absorbiert. Die Wäscheschutzfolie 3 ist gegenüber Flüssigkeiten undurchlässig und besteht z.B. aus Polyätylen oder Polypropylen. Die Windel 1 weist zwischem hinteren Bund 7, der mit Vlieselementen 8 versehen ist, und dem vorderen Bund 9 einen Beinausschnitt 10 auf. Der Beinausschnitt 10 kann jede beliebige Form aufweisen und die Erfindung ist nicht auf die in den Figuren dargestellten Formen beschränkt. Schließlich ist eine Seitenklappe 11 erkennbar, die entlang ihrer einen Längskante 12 mit der Vliesstoffabdeckung 5 z.B. über eine Verklebung 13 verbunden ist. Die gegenüberliegende Längskante 14 der Seitenklappe 11 überragt die Längskante 15 der Windel 1 und somit auch den Beinausschnitt 10. Bei dem in Fig. 1 wiedergegebenen Ausführungsbeispiel ist die Seitenklappe 11 trapezförmig ausgebildet, wobei die schmälere Längskante 14 die üblicherweise vom Rand der Wäscheschutzfolie 3 und vom Rand der Vliesstoffabdeckung 4 gebildete Dichtungsleite 16 überragt.

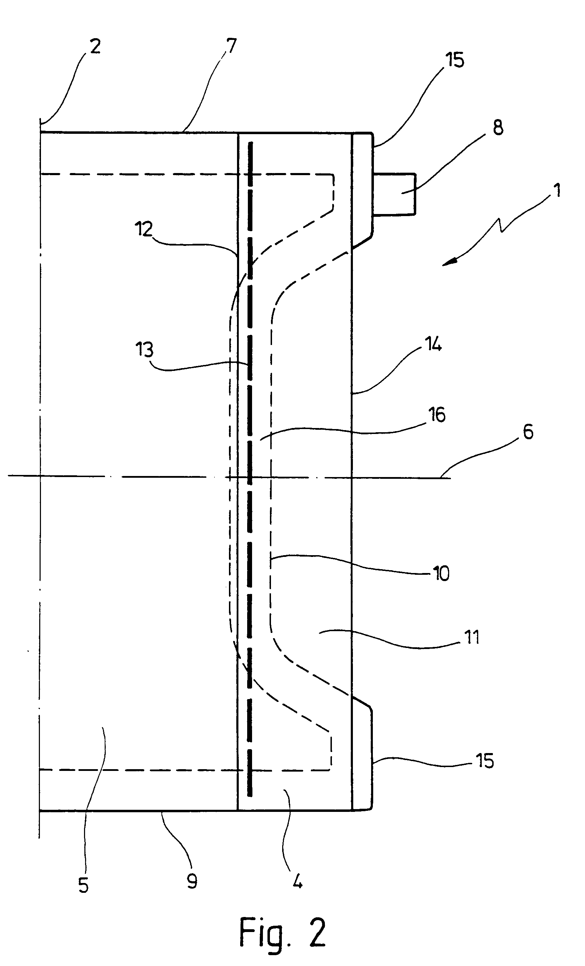

Bei dem in Fig. 2 wiedergegebenen Ausführungsbeispiel weist die Seitenklappe 11 eine Rechteckform auf und erstreckt sich vom hinteren Bund 7 bis zum vorderen Bund 9, wobei auch bei dieser Ausführungsform die eine Längskante 12 über eine Klebverbindung 13 mit der Vliesstoffabdeckung 3 verbunden ist und die andere Längskante 14 den Beinausschnitt 10 überragt. Im Bereich des Beinausschnitts 10 befindet sich bei diesem, in Fig. 2 wiedergegebenen Ausführungsbeispiel, die Verklebung 13 außerhalb des Saugkörpers 5, wohingegen die Verklebung 13 beim Ausführungsbeispiel der Fig. 1 innerhalb des Saugkörpers 5 liegt.

Bei dem in Fig. 3 wiedergegebenen Querschnitt III-III gemäß Fig. 1 ist die an der Unterseite der Windel 1 liegende Wäscheschutzfolie, die die Oberseite bildende Vliesstoffabdeckung 4 und der dazwischenliegende Saugkörper 5 erkennbar. Die Folie 3 und die Abdeckung 4 bilden an ihrem Rand die Dichtungsleiste 16, die ein Auslaufen der Windel 1 verhindern soll. Über der Dichtungsleiste 16 befindet sich die Seitenklappe 11, deren Längskante 12 mit der Vliesstoffabdeckung 4 oberhalb des Saugkörpers 5 verklebt ist und deren Längskante 14 die Dichtungsleiste 16 frei überragt. Mit dieser Seitenklappe 11 kann nunmehr Flüssigkeit aus der Windel über die Dichtungsleiste 16 hinausgeleitet werden. Wird der die Windel überragende Teil der Seitenklappe 11 von Luft durchströmt, so verdunstet ein Teil der absorbierten Flüssigkeit und die Seitenklappe 11 wird aufgrund der Verdampfungsenthalpie kalt, was von der inkontinenten Person als unangenehm empfunden wird. Die Seitenklappen haben die Aufgabe eines Flüssigkeitsindikators.

Zum besseren Anliegen am Oberschenkel des Benutzers können die Seitenklappen 11 mit einem oder mehreren elastischen Elementen versehen sein.

## Patentansprüche

1. Wegwerfwindel (1) mit einer gegenüber Flüssigkeit undurchlässigen Wäscheschutzfolie (3), einer gegenüber Flüssigkeit durchlässigen Vliesstoffabdeckung (4) und einem zwischen der Wascheschutzfolie (3) und der Vliesstoffabdeckung (4) sich befindenden Saugkörper (5), sowie die Außenkontur bildenden Dichtungsleisten (16),
**dadurch gekennzeichnet,**
daß für die Außenkontur der Dichtungsleisten (16) seitlich zumindest teilweise überragende, hydrophile Seitenklappen (11) vorgesehen sind, die mit der gegenüber Flüssigkeit durchlässigen Vliesstoffabdeckung (4) verbunden sind.

2. Wegwerfwindel nach Anspruch 1, dadurch gekennzeichnet, daß die hydrophilen Seitenklappen (11) im Bereich der Beinausschnitte (10) vorgesehen sind und die Außenkontur der Dichtungsleisten (16) überragen.

3. Wegwerfwindel nach Anspruch 1, dadurch gekenn-zeichnet, daß die Seitenklappen (11) sich über die ganze Länge der Windel (1) erstrecken.

4. Wegwerfwindel nach einem der vorhergehenden Ansprüche, dadurch gekennzeichnet, daß die Seitenklappen (11) in einem Bereich mit der Vliesstoffabdeckung (4) verbunden sind, in dem diese den Saugkörper (5) überdeckt.

5. Wegwerfwindel nach einem der vorhergehenden Ansprüche, dadurch gekennzeichnet, daß die Seitenklappen (11) gegenüber Flüssigkeiten eine Dochtwirkung besitzen und absorbierend sind.

6. Wegwerfwindel nach einen der vorhergehenden Ansprüche, dadurch gekennzeichnet, daß die Seitenklappen (11) mit einem oder mehreren elastischen Elementen versehen sind.

7. Wegwerfwindel nach einem der vorhergehenden Ansprüche, dadurch gekennzeichnet, daß die Seitenklappen (11) bei an dem Körper anliegender Windel (1) sich von der Windel (1) wegerstrecken.

8. Wegwerfwindel nach einem der vorhergehenden Ansprüche, dadurch gekennzeichnet, daß die Seitenklappen (11) bei an dem Körper angelegter Windel (1) an den Innenseiten der Schenkel des Trägers anliegen.

9. Wegwerfwindel nach einem der vorhergehenden Ansprüche, dadurch gekennzeichnet, daß die Seitenklappen (11) aus Viskose bestehen.

10. Wegwerfwindel nach einem der vorhergehenden Ansprüche, dadurch gekennzeichnet, daß die Seitenklappen (11) auf die Vliesstoffabdeckung (4) aufgeklebt sind.

11. Wegwerfwindel nach einem der vorhergehenden Ansprüche, dadurch gekennzeichnet, daß die Seitenklappen (11) mit der Vliesstoffabdeckung (4) einstückig sind.

## Claims

1. Disposable diaper (1) having a protective film (3) that is impermeable to liquids, a non-woven fabric cover (4) that is permeable to liquids, and an absorbent body (5) arranged between the protective film (3) and the non-woven fabric cover (4), and sealing strips (16) forming the outer contour,
**characterized in that**
hydrophilous lateral flaps (11) are provided that project laterally over at least part of the outer contour of the sealing strips (16) and that are connected with the non-woven fabric cover (4) the latter being permeable to liquids.

2. Disposable diaper according to claim 1, characterized in that the hydrophilous lateral flaps (11) are provided in the area of the leg opening (10) and project beyond the outer contour of the sealing strips (16).

3. Disposable diaper according to claim 1, characterized in that the lateral flaps (11) extend over the full length of the diaper (1).

4. Disposable diaper according to any of the preceding claims, characterized in that the lateral flaps (11) are connected with the non-woven fabric cover (4) in an area in which it overlaps the absorbent body (5).

5. Disposable diaper according to any of the preceding claims, characterized in that the lateral flaps (11) have a wick-like effect on liquids and are of an absorbent nature.

6. Disposable diaper according to any of the preceding claims, characterized in that the lateral flaps (11) are provided with one or more elastic elements.

7. Disposable diaper according to any of the preceding claims, characterized in that when the diaper (1) is applied to the body, the lateral flaps (11) extend away from the diaper (1).

8. Disposable diaper according to any of the preceding claims, characterized in that when the diaper (1) is applied to the body, the lateral flaps (11) apply themselves to the insides of the legs of the person wearing the diaper.

9. Disposable diaper according to any of the preceding claims, characterized in that the lateral flaps (11) consist of viscose.

10. Disposable diaper according to any of the preceding claims, characterized in that the lateral flaps (11) are fixed on the non-woven fabric cover (4) by an adhesive.

11. Disposable diaper according to any of the preceding claims, characterized in that the lateral flaps (11) are formed integrally with the non-woven fabric cover (4).

## Revendications

1. Couche à jeter (1) avec une pellicule (3) imperméable pour protéger le linge, un recouvrement (4) en nappe de fibres perméable aux liquides et un corps absorbant (5) situé entre la pellicule (3) de protection du linge et le recouvrement en nappe de fibres (4), ainsi qu'une garniture d'étanchement (16) formant le bord extérieur,
**caractérisée en ce que**
des volets hydrophiles latéraux (11) faisant saillie latéralement au moins en partie sont prévus pour le bord extérieur des garnitures d'étanchement (16), ces volets étant reliés avec le recouvrement en nappe de fibres (4) perméable aux liquides.

2. Couche à jeter selon la revendication 1, caractérisée en ce que les volets latéraux hydrophiles (11) sont prévus dans la zone des découpures (10) pour les jambes et dépassent le bord extérieur des garnitures d'étanchement (16).

3. Couche à jeter selon la revendication 1, caractérisée en ce que les volets latéraux (11) s'étendent sur toute la longueur de la couche (1).

4. Couche à jeter selon l'une des revendications précédentes, caractérisée en ce que les volets latéraux (11) sont reliés avec le recouvrement en nappe de fibres (4) dans une zone dans laquelle ce dernier recouvre le corps absorbant (5).

5. Couche à jeter selon l'une des revendications précédentes, caractérisée en ce que les volets latéraux (11) présentent un effet d'étanchement contre les liquides et sont absorbants.

6. Couche à jeter selon l'une des revendications précédentes, caractérisée en ce que les volets latéraux (11) sont munis d'un ou de plusieurs éléments élastiques.

7. Couche à jeter selon l'une des revendications précédentes, caractérisée en ce que les volets latéraux (11) s'étendent vers l'extérieur de la couche (1) si la couche (1) est appliquée au corps.

8. Couche à jeter selon l'une des revendications précédentes, caractérisée en ce que les volets latéraux (11) reposent sur les faces intérieures des cuisses du porteur si la couche (1) est appliquée au corps.

9. Couche à jeter selon l'une des revendications précédentes, caractérisée en ce que les volets latéraux (11) sont en viscose.

10. Couche à jeter selon l'une des revendications précédentes, caractérisée en ce que les volets latéraux (11) sont collés sur le recouvrement en nappe de fibres (4).

11. Couche à jeter selon l'une des revendications précédentes, caractérisée en ce que les volets latéraux (11) forment une pièce avec le recouvrement en nappe de fibres (4).
